# EUROPEAN PATENT APPLICATION

(11) **EP 2 168 476 A1**
(43) Date of publication of application: **31.03.2010**
(21) Application number: 09012119.5
(22) Date of filing: 23.09.2009
(51) Int. Cl.: A61B 5/022

(54) **Blood pressure meter**

(30) Priority: 25.09.2008 JP 2008245709
(71) Applicant: Panasonic Electric Works Co., Ltd., Kadoma-shi Osaka 571-8686 (JP)
(72) Inventor: Fumuro, Shinichi, Kadoma-shi Osaka 571-8686 (JP); Mizuuchi, Akihiro, Kadoma-shi Osaka 571-8686 (JP); Kojima, Takeshi, Kadoma-shi Osaka 571-8686 (JP); Kanbayashi, Tadashi, Kadoma-shi Osaka 571-8686 (JP); Yuasa, Tsuyoshi, Kadoma-shi Osaka 571-8686 (JP); Oguri, Kazuya, Kadoma-shi Osaka 571-8686 (JP)
(74) Representative: Müller-Boré & Partner Patentanwälte

(57) **Abstract**

A blood pressure meter includes: a cuff; a case main body that has a cuff storing recess capable of storing the cuff at a back-side position of the case main body; a main block that is provided in the case main body at a front-side position and that has a display unit and an operation unit; a cover that is joined to a portion on the back side of the case main body to open and close freely by way of a hinge and that can cover the upper portion the case main body; and a lift-up portion that can lift the cuff upward from the cuff storing recess.

## Description

### Background of the Invention

### Field of the Invention

The present invention relates to a blood pressure meter.

### Description of the Background Art

JP 8-317911 A discloses a blood pressure meter comprising a case main body; a cover (lid) mounted on the case main body to open and close freely by way of a hinge; a finger cuff partially fixed to the inner side of the cover; and a finger cuff storing portion provided in the case main body.

In this blood pressure meter, the finger cuff is partially fixed to the inner side of the cover. Therefore, the finger cuff is removed from the finger cuff storing portion upon opening of the cover, and is stored in the finger cuff storing portion upon closing of the cover. The size of the finger cuff is small, and hence it is possible to remove the finger cuff from the finger cuff storing portion at the same time that the cover is opened, and to measure blood pressure by inserting a finger into the finger cuff, with the latter fixed to the cover.

Cuffs that are wrapped around an arm, however, are larger in size, and are hence harder to remove from a cuff storing portion in the same way as a finger cuff is. With the cuff partially fixed to the cover, it is difficult to remove the cuff from the cuff storing portion at the same time that the cover is opened. It is likewise difficult to insert the arm in the cuff, or to measure blood pressure by wrapping the cuff around the arm, with the cuff partially fixed to the cover.

Removing the cuff could be made easier by raising the bottom of the cuff storing portion provided at a position on the back side of the case main body (at a position on the hinge side), but doing so entails an overall larger case main body, which precludes achieving a compact blood pressure meter. Alternatively, the cuff storing portion could be formed at a front-side position of the case main body (at a position on the side opposite the hinge), but this approach is poor in design, and results moreover in a larger front-side portion of the case main body, which precludes achieving a compact blood pressure meter.

### Summary of the Invention

It is thus an object of the present invention to provide a blood pressure meter from which a cuff can be removed easily even when a cuff storing recess is provided at a position on the hinge side of a case main body, without making the blood pressure meter less compact as a result.

The blood pressure meter of the present invention includes: a cuff; a case main body that has a cuff storing recess capable of storing the cuff; a main block that is provided in the case main body and that has a display unit and an operation unit; and a cover joined to the case main body to open and close freely by way of a hinge and that can cover an upper portion of at least the cuff storing recess of the case main body. In the case main body, the cuff storing recess is provided at a position on a hinge side, and the main block is provided at a position on a side opposite the hinge. The blood pressure meter further comprises a lift-up portion capable of lifting the cuff upward from the cuff storing recess in response to a cover opening operation, or after the cover opening operation.

These and other objects, features and advantages of the present invention will become more apparent upon reading of the following detailed description along with the accompanied drawings.

### Brief Description of the Drawings

Fig. 1 is a perspective-view diagram illustrating a blood pressure meter according to a first embodiment of the present invention, with a cover closed;
Fig. 2 is a perspective-view diagram illustrating the blood pressure meter according to the first embodiment of the present invention, with the cover opened;
Fig. 3 is a schematic side-view diagram illustrating the blood pressure meter of the first embodiment; and
Fig. 4 is a schematic side-view diagram illustrating a blood pressure meter of a second embodiment.

### Description of the Preferred Embodiments

Embodiments of the present invention will be explained in detail below with reference to accompanying drawings.

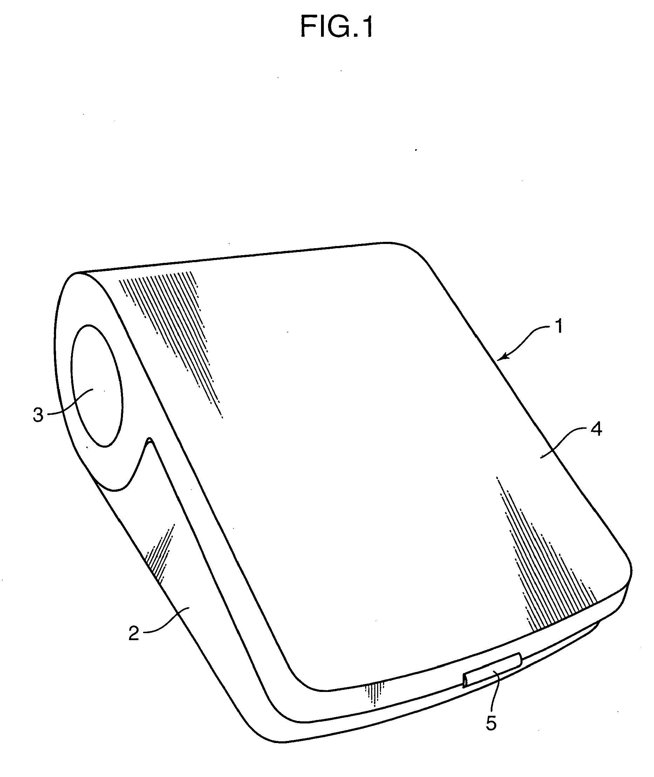

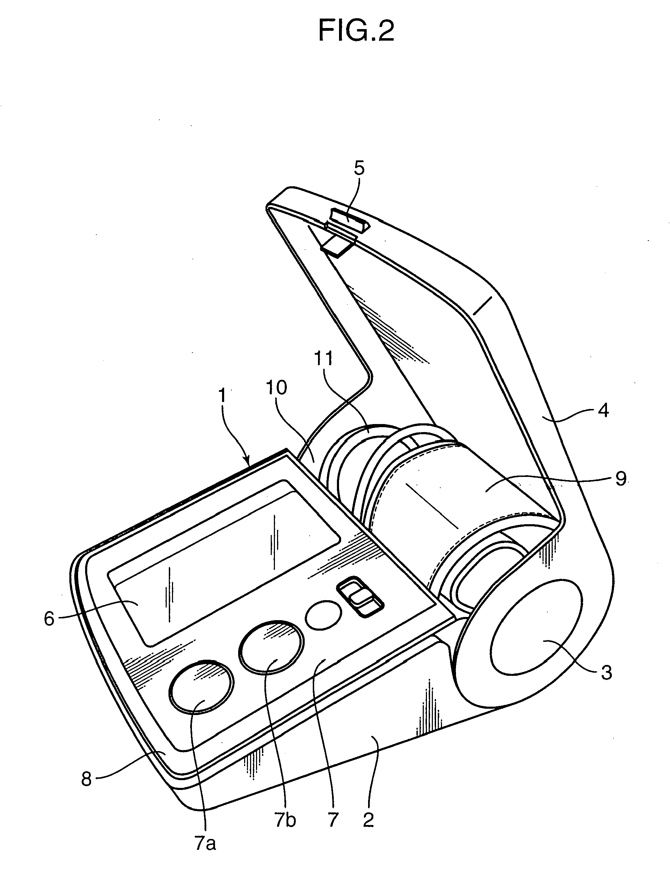

Fig. 1 illustrates a blood pressure meter 1 of a first embodiment with a cover 4 in a closed state. Fig. 2 illustrates the blood pressure meter 1 of the first embodiment with the cover 4 in an open state.

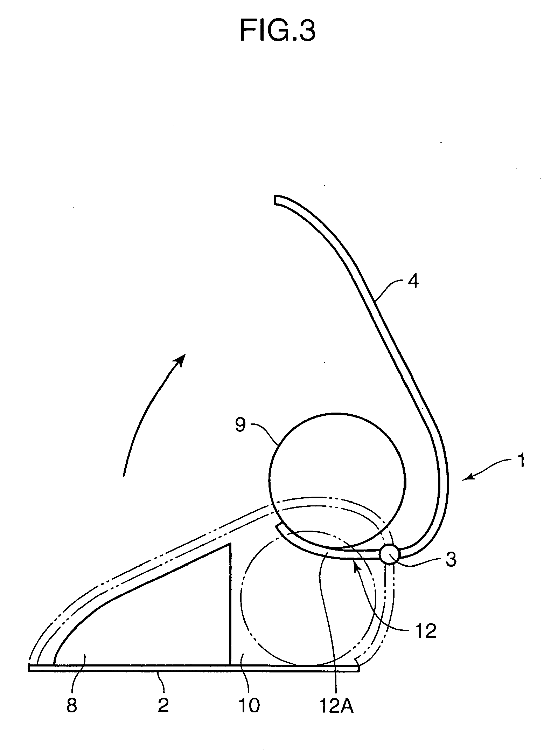

The blood pressure meter 1 comprises a case main body 2 and the cover 4, which is joined to a portion on the back side of the case main body 2 to open and close freely by way of a hinge portion 3 (see Fig. 3). In the present embodiment, the cover 4 covers the entire upper portion of the case main body 2, but may cover just the upper portion of a below-described cuff storing recess 10.

In the closed state of Fig. 1, the cover 4 is locked to the case main body 2 by way of a locking part 5, to prevent accidental opening. When the locking part 5 is released manually, the cover 4 rises almost perpendicularly relative to the case main body 2, as illustrated in Fig. 2.

A main block 8, having an operation unit 7 provided with, for instance, a display unit (liquid crystal display) 6, a power supply switch 7a, a pressurization start switch 7b and the like, is provided at a front-side position of the case main body 2 (at a position opposite the hinge portion 3). Inside the main block 8 there are housed, for instance, a control unit as well as a pump, not shown, capable of supplying air to, and evacuating air out of, a below-described cuff (band-like member) 9.

The cuff storing recess 10, in which the cuff 9 can be stored, is formed at a position on the back side of the case main body 2 (at a position on the side of the hinge portion 3). The cuff 9 and the pump are connected by way of a flexible tube 11 through which air passes. The tube 11 extends from the pump in the main block 8 to outside the main block 8, through a hole, not shown, in the main block 8. The tube 11 is long enough so as to allow the cuff 9 to reach a position where it can be easily wrapped around an arm.

During use, the cuff 9 is unrolled horizontally, and is then wrapped around the arm. The wrapped cuff 9 is fixed to the arm by way of, for instance, a surface fastener (hook-and-loop fastener) attached to the end of the cuff 9.

The cuff 9 is stored, for instance, rolled up as a roll (see Fig. 3). The rolled-up cuff 9 can be stored, together with the tube 11, in the cuff storing recess 10, from above, at a position on the back side of the case main body 2, as illustrated in Fig. 2.

The blood pressure meter 1 comprises a lift-up portion 12 capable of lifting up (raising) the cuff 9 up from the cuff storing recess 10 when the cover 4 is open.

The lift-up portion 12 in the first embodiment is a rib 12A integrally provided in the cover 4 at the hinge portion 3, as illustrated in Fig. 3. The rib 12A extends from the hinge portion 3 in the direction opposite to the direction in which the cover 4 extends from the hinge portion 3. The rib 12A is formed to a circular arc shape substantially identical to the outer peripheral face of the rolled cuff 9. The rib 12A comprises, for instance, a plate-like or a rod-like member.

In the first embodiment, the cover 4 and the rib 12A can be formed as an integral body, out of a material such as a resin, a metal or the like. In such an integrally formed body, the cover 4 constitutes the area extending from the portion at which the hinge portion 3 is attached to the formed body, up to the leading end (front end) of the formed body, whereas the rib 12A constitutes the area extending from the portion at which the hinge portion 3 is attached to the formed body, up to the rear end of the formed body. The leading end of the formed body refers herein to the end of the formed body positioned at the front side when the case main body 2 is covered by the cover 4 through closing of the latter. The rear end of the formed body refers to the lower end of the formed body positioned at the rear side when the cover 4 is closed.

As illustrated in Figs. 2 and 3, the case main body 2 comprises a bottom plate and a pair of side walls extending upwards from both the right and left side edges of the bottom plate. The hinge portion 3 is provided at a back side portion of the side walls of the case main body 2. The cover 4 pivots relative to the case main body 2 about a pin of the hinge portion 3.

In Fig. 3, the solid line depicts a state in which the cuff 9 is scooped up upon opening of the cover 4, while the double-dotted line depicts a state in which the cuff 9 is stored in the cuff storing recess 10 upon closing of the cover 4. In the stored state, the hinge portion 3 is positioned at a height similar to or higher than that of the center of the rolled-up cuff 9 (center of the double-dotted line circle in Fig. 3).

With the cuff 9 stored in the cuff storing recess 10 at a position on the back side of the case main body 2, the rib (lift-up portion) 12A of the first embodiment scoops up the cuff 9 in response to the opening operation of the cover 4, whereby the cuff 9 can be lifted up from the cuff storing recess 10. The rib 12A points in a substantially horizontal direction, as illustrated in Fig. 3, when the cuff 9 is thus lifted up through opening of the cover 4.

The cuff 9 lifted up from the cuff storing recess 10 can thus be easily extracted from between the main block 8 and the cover 4. To store the cuff 9, the latter is placed on the rib 12A and the cover 4 is closed. As a result of this operation, the cuff 9 becomes stored in the cuff storing recess 10 while being pressed down by the rear face of the cover 4.

When the cuff 9 is stored in the cuff storing recess 10 through closing of the cover 4, the rib 12A becomes disposed extending downward from the hinge portion 3. The rib 12A constitutes then the rear-side wall of the blood pressure meter 1. In this state, the rib 12A constitutes as well the rear face of the cuff storing recess 10, as illustrated in Fig. 3.

The blood pressure meter 1 of the first embodiment has thus a structure whereby the rib 12A scoops the cuff 9 up from the cuff storing recess 10, and in consequence the case main body 2 is not large. This affords a compact blood pressure meter 1. The rib 12A, moreover, is structured so as to be formed integrally with the cover 4. Costs can be kept therefore low thanks to such a simple structure. Also, the lift-up portion 12 of the first embodiment can be provided in already-existing blood pressure meters by simply replacing the cover.

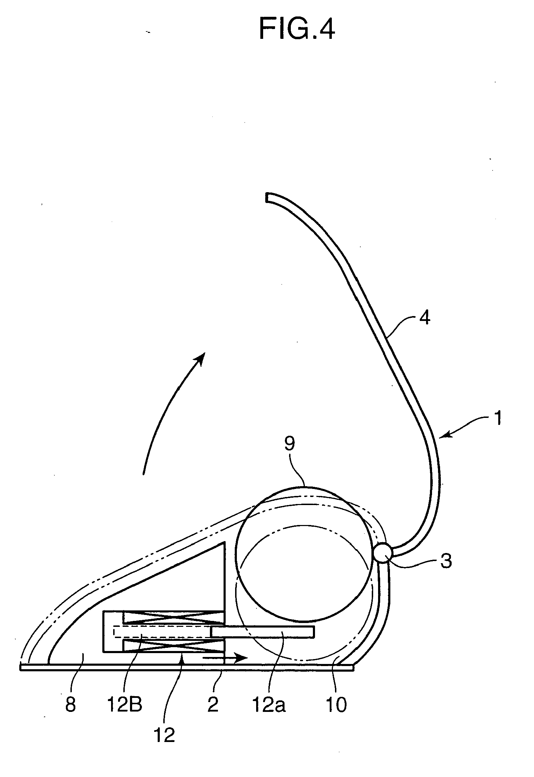

As illustrated in Fig. 4, the lift-up portion 12 of the second embodiment is a solenoid (raising mechanism) 12B. The solenoid 12B comprises a solenoid main body provided in the main block 8 of the case main body 2, and a plunger 12a that can protrude from the solenoid main body towards a position at the back side. When the solenoid 12B is magnetized upon turning-on of the power source through pressing of the power supply switch (actuating operation unit) 7a, the plunger 12a protrudes out of the main block 8 in a direction towards the bottom side of the cuff storing recess 10, through a through-hole (not shown) provided in the main block 8. A dedicated switch for actuating the lift-up portion 12 may also be provided as the actuating operation unit, separately from the supply switch 7a.

In Fig. 4, the solid line depicts a state in which the cover 4 is open and the cuff 9 is raised, and the double-dotted line depicts a state in which the cuff 9 is stored in the cuff storing recess 10 and the cover 4 is closed. In the stored state, the plunger 12a is provided at a position lower than the center of the rolled-up cuff 9 (center of the double-dotted line circle in Fig. 3), in such a manner so as to be able to protrude at a position lower than the center of the cuff 9.

In the blood pressure meter 1 of the second embodiment, the cuff 9 does not rise by simply opening of the cover 4 when the cuff 9 is stored in the cuff storing recess 10 at a position on the back side of the case main body 2.

Herein, the plunger 12a protrudes as a result of a separate operation after opening of the cover 4, namely as a result of magnetization of the solenoid 12B upon turning-on of the power source through pressing of the power supply switch (actuating operation unit) 7a. The plunger 12a can lift up the cuff 9 by pushing the latter up.

To store the cuff 9, the cuff 9 is placed on the plunger 12a, and then the power supply is turned off by pressing the power supply switch 7a, whereby the solenoid 12B is demagnetized and the plunger 12a returns to the position prior to magnetization (the plunger 12a is inserted in the solenoid main body). The position of the plunger 12a is depicted by the dashed line in Fig. 4. Closing next the cover 4 causes the cuff 9 to be stored in the cuff storing recess 10, as the rear face of the cover 4 pushes also against the cuff 9.

A better design is thus achieved by having the cuff 9 being lifted up through an operation separate from the operation of opening the cover 4, so that the cuff 9 does not rise when it is simply desired to look at past measurement data on the display unit 6 of the main block 8, with the cover 4 open.

A summary of the above embodiments follows next.

In a blood pressure meter using a cuff large enough to be wrapped around an arm, there may provided a main block having a display unit and/or an operation unit at a position on the front side of a case main body (position on a side opposite a hinge), while a cuff storing recess, in which the rolled-up cuff can be stored, is formed at a position on the back side of the case main body (position on the side of the hinge). The upper portion of at least the cuff storing recess of the case main body is covered by a cover that is joined to the a portion on the back side of the case main body to open and close freely. The cover protects the stored cuff against dust.

However, in blood pressure meters where a cuff storing recess is formed at a position on the back side of case main body, the main block and the cover are hindrances that make it difficult to remove the cuff from the cuff storing recess.

Therefore, the blood pressure meter 1 in the above embodiments is provided with a lift-up portion 12 that is capable of lifting the cuff 9 up from the cuff storing recess 10 in response to the opening operation of the cover 4, or after the cover 4 is opened. In a state where the cuff 9 is stored in the cuff storing recess 10 at a position on the back side of the case main body 2, thus, the lift-up portion 12 lifts the cuff 9 up from the cuff storing recess 10 in response to the opening of the cover 4, or after the cover 4 is open. This makes it easier to extract the cuff 9 between the main block 8 and the cover 4.

In the first embodiment, the lift-up portion 12 is a rib 12A capable of scooping up the cuff 9 in response to the opening operation of the cover 4. The structure of the lift-up portion 12 of the first embodiment is thus such that the rib 12A scoops the cuff 9 up from the cuff storing recess 10, and in consequence the case main body 2 is not large. This affords a compact blood pressure meter 1. The rib 12A, moreover, is structured so as to be formed integrally with the cover 4. Costs can be kept therefore low thanks to such a simple structure. Also, the lift-up portion 12 of the first embodiment can be provided in already-existing blood pressure meters by simply replacing the cover.

The blood pressure meter 1 in the second embodiment has an actuating operation unit that actuates the lift-up portion 12. Once the cover 4 is open, operation of the actuating operation unit causes the lift-up portion 12 to lift the cuff 9 up from the cuff storing recess 10.

A better design is thus achieved by having the cuff 9 being lifted up through an operation separate from the operation of opening the cover 4, so that the cuff 9 does not rise when it is simply desired to look at past measurement data on the display unit 6 of the main block 8, with the cover 4 open. Because of this, the appearance of the meter in a state, where the cover 4 is opened, can be improved.

Specifically, the lift-up portion 12 in the second embodiment is a solenoid 12B that is actuated through the operation of the actuating operation unit. The solenoid 12B has a plunger 12a that can protrude towards the cuff storing recess 10 as a result of an operation of the actuating operation unit. The plunger 12a is constructed so as to push up the cuff 9 stored in the cuff storing recess 10 as the plunger 12a protrudes towards the cuff storing recess 10.

The second embodiment has been explained based on an example in which an electric solenoid 12B is used as the lift-up portion 12. The embodiment, however, is not limited thereto. For instance, the blood pressure meter may comprise a lever-shaped member having a push-up portion (lift-up portion) disposed inside the cuff storing recess 10, and an actuating operation lever (actuating operation unit), integral with the push-up portion, disposed outside the case main body 2. In this case, the push-up portion can lift the cuff 9 up by pushing the latter up through an operation wherein the actuating operation lever disposed outside the case main body 2 is pressed down.

This application is based on Japanese Patent application serial No. 2008-245709 filed in Japan Patent Office on September 25, 2008, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. A blood pressure meter, comprising:
a cuff;
a case main body that has a cuff storing recess capable of storing the cuff;
a main block that is provided in the case main body and that has a display unit and an operation unit; and
a cover that is joined to the case main body to open and close freely by way of a hinge and that can cover an upper portion of at least the cuff storing recess of the case main body,
in the case main body, the cuff storing recess being provided at a position on a hinge side, and the main block being provided at a position on a side opposite the hinge,
the blood pressure meter further comprising a lift-up portion capable of lifting the cuff upward from the cuff storing recess in response to a cover opening operation, or after the cover opening operation.

2. The blood pressure meter according to claim 1, wherein the lift-up portion is a rib capable of scooping the cuff up in response to the cover opening operation.

3. The blood pressure meter according to claim 1 or 2, comprising an actuating operation unit that actuates the lift-up portion, wherein
after the cover opening operation, an operation of the actuating operation unit causes the lift-up portion to lift the cuff upward from the cuff storing recess.

4. The blood pressure meter according to claim 3, wherein the lift-up portion is a solenoid to be actuated through the operation of the actuating operation unit.

5. The blood pressure meter according to claim 4, wherein
the solenoid has a plunger capable of protruding towards the cuff storing recess so as to push up the cuff stored in the cuff storing recess according to the operation of the actuating operation unit.
